# EUROPÄISCHE PATENTSCHRIFT

(11) **EP 0 686 697 B1**
(45) Veröffentlichungstag und Bekanntmachung des Hinweises auf die Patenterteilung: **14.04.2004**
(21) Anmeldenummer: 95250109.6
(22) Anmeldetag: 08.05.1995
(51) Int. Cl.: C12N 15/89, C12N 5/10, C12N 13/00, C12N 15/18, C12N 15/19

(54) **Methode zur Anreicherung von Zellen, die durch ballistischen Transfer modifiziert wurden**
Method for the enrichment of cells modified by ballistic transfer
Méthode d'enrichissement de cellules modifiées par transfert balistique

(30) Priorität: 09.05.1994 DE 4416784
(43) Veröffentlichungstag der Anmeldung: 13.12.1995
(73) Patentinhaber: Mologen AG, 14195 Berlin (DE)
(72) Erfinder: Wittig, Burghardt, Prof. Dr., D-14197 Berlin (DE); Schroff, Matthias, D-30853 Langenhagen (DE)
(74) Vertreter: Omsels, Hermann-Josef

(56) Entgegenhaltungen:
- EP-A- 0 486 233
- WO-A-90/07380
- WO-A-91/07487
- J IMMUNOL METHODS, OCT 15 1993, 165 (2) P149-56, NETHERLANDS, XP000571662 BURKHOLDER JK ET AL: "Rapid transgene expression in lymphocyte and macrophage primary cultures after particle bombardment-mediated gene transfer."

## Beschreibung

Viele Fragestellungen und Anwendungen moderner molekularbiologischer Methoden erfordern das Einschleusen von Material in Zellen. Besondere Bedeutung kommt dabei vor allem dem Einbringen von Nucleinsäurekonstrukten zu, die das Genom der Zellen verändern oder seine Expression modifizieren sollen. Die heute dafür zur Verfügung stehenden Verfahren gehen u.a. von einem durch Viren oder Liposomen vermittelten Transport, der Permeabilisierung der Zellen z.B. durch Elektroporation und anschließender Diffusion einer im Medium enthaltenden Substanz in die Zelle oder direktem mechanischen Transport der Substanz aus. Eine ausführliche Übersicht des gegenwärtigen Standes der Technik wird in *Methods in Enzymology*, Band 217 (Academic Press, San Diego, CA, 1993), Seiten 461-655, gegeben.

Bei der Methode des direkten mechanischen Transports in die Zelle kann entweder eine die zu transportierende Substanz enthaltende Lösung in die Zelle injiziert werden (DE PS 3718066), oder die Substanz in fester Form, an einen Träger adsorbiert oder in Lösung, auf die Zelle beschleunigt werden, so daß sie die Zellwand durchschlägt und in die Zelle eindringt (sog. Transfer"), ohne daß die Zelle durch das Eindringen dieses Mikroprojektiles untergeht (J. Immunol. Methods 165,1993, p 149-156).

Bis auf die Mikroinjektion leiden diese Verfahren unter der zum Teil sehr geringen Effizienz des Transports in die Zelle. Im Falle der Mikroinjektion ist die Effizienz, ausgedrückt in Anteil der erfolgreich behandelten Zellen an der Gesamtzahl behandelter Zellen, zwar sehr groß, doch ist die Methode sehr zeit- und arbeitsaufwendig, so daß nur kleine Mengen von Zellen behandelt werden können.

Der ballistische Transfer ermöglicht prinzipiell eine unkompliziertere Art des Substanztransports in Zellen. Die Methode wurde bisher vor allem bei Pflanzenzellen angewendet (siehe *Current Opinion in Biotechnology* , Band 4, Seiten 135- 141, 1993), da für diese oftmals keine geeigneten Alternativen bereitstehen, doch auch bei Säugerzellen (Fitzpatrick-McElligott, *Biotechnology (New York)* , Band 10, S. 1036-1040 (1992); WO 9107487) und Prokaryonten (Smith et. al., *Journal of General Microbiology* , Band 138, S. 138 ff.) war sie erfolgreich. Es lassen sich in einem Arbeitsschritt viele Tausende bis Millionen Zellen behandeln. Dabei wird die zu transportierende Substanz an Mikroprojektile adsorbiert, und diese werden durch Zünden einer explosiven Ladung (EP 0 331 855; EP 0 397 413), eine durch Druckdifferenz herbeigeführte Gasstoßwelle (WO 91/18991) oder eine durch elektrische Entladung herbeigeführte Druckwelle (WO 91/00359) auf die Zielzellen beschleunigt. Ebenfalls bekannt ist eine Methode, die sich der Injektion mittels eines Aerosolstrahls bedient (WO 91/00915). Siehe auch: EP 0397413; GB 9018892.1; US 5066587; WO 91/ 02071; WO 91/11526.

Beim ballistischen Transfer wird nur ein Teil der Zielzellen getroffen. Verdichtet man die auftreffende Garbe der Mikroprojektile derart, daß praktisch alle Zielzellen getroffen werden, ist mit einer sehr hohen Verlustrate zu rechnen, da eine zu große Zahl von Treffern an einer einzelnen Zelle zum einzelnen Zelle zum Untergang derselben führen wird. Ein Kompromiß zwischen hoher Trefferrate und hoher Überlebensrate der Zielzellen wird also immer dazu führen, daß ein Teil der Zielzellen unbehandelt bleibt.

In den meisten Fällen wird es aber erwünscht sein, nur die erfolgreich behandelten Zellen zu isolieren. Ist die eingeschleuste Substanz etwa eine Nucleinsäure, die das Genom der Zelle dauerhaft oder transient verändert, oder in dessen Expression eingreift, so müssen bei herkömmlichen Verfahren die veränderten von den nicht veränderten Zellen durch Weiterzucht oder Selektion getrennt werden, was bei vielen Anwendungen die Brauchbarkeit der Methode einschränkt. Bei der *Ex vivo-* Behandlung von Tumorzellen etwa ist es unabdingbar, nur die erfolgreich manipulierten Zellen in den Patienten zurückzugeben.

Ein Verfahren, das schonend und in kürzester Zeit die getroffenen Zellen von den nicht getroffenen Zellen abtrennt, wäre demnach für die Verwendbarkeit des ballistischen Transfers in der Biotechnologie und Medizin von großem Vorteil.

Seit einiger Zeit sind in der Biochemie Arbeitsverfahren bekannt, die die Abtrennung bestimmter Anteile von Zell- oder Partikelgemischen dadurch ermöglichen, daß der abzutrennende Bestandteil über spezifische oder unspezifische Wechselwirkungen an in dem Gemisch befindliche ferromagnetische Partikel gebunden wird. Nach Anlegen eines Magnetfeldes werden die Partikel mit den an ihnen gebundenen abzutrennenden Bestandteilen im Magnetfeld festgehalten, während der Rest des Gemisches entfernt werden kann. Die abzutrennende Substanz kann nach Entfernen des angelegten Feldes, gegebenenfalls nach Abtrennung der Magnetpartikel, weiterbehandelt werden. Die Bindung von Zellen an die Magnetpartikel wird dabei vor allem dadurch erreicht, daß biologische Moleküle an die Magnetpartikel kovalent gebunden werden, die ihrerseits starke spezifische Bindungen mit auf der Zelloberfläche gebundenen Molekülen eingehen. Auf diese Weise lassen sich z.B. verschiedene Leukozytenlinien nach ihrer unterschiedlichen Ausstattung mit CD-Oberflächenmarkern isolieren.

Die verwendeten Systeme zum Abtrennen unterscheiden sich dabei vor allem in der Größe der verwendeteten Partikel und der Stärke des angelegten Feldes. Größere ferromagnetische Partikel (>0.5 µm) werden von verhältnismäßig schwachen, von einem einfachen Permanentmagneten erzeugten Feld über mehrere Milimeter hinweg angezogen und zurückgehalten. Diese Partikelgrößen bringen aber den Nachteil mit sich, daß die Partikel nach Entfernen des Magnetfeldes eine gewisse Restmagnetisierung behalten, da sie größer sind als die Weis'schen Bezirke, die das ferrromagnetische Verhalten der Partikel erzeugen, und sich bei gleichsinniger Anordnung der in ihnen enthaltenen Weis'schen Bezirke so verhalten wie kleine Permanentmagneten. Durch die dadurch entstehende gegenseitige Anziehung neigen die Partikel zur Aggregation und sedimentieren schneller.

Reduziert man die Größe der Partikel unter einen Schwellenwert, verhalten sich die Partikel "superferromagnetisch", d.h. es bleibt keine Restmagnetisierung nach Entfernen des Feldes zurück. Durch die fehlende Restmagnetisierung und die geringere Partikelgröße halten sich die Partikel in kolloidaler Lösung, statt zu sedimentieren, und eignen sich dadurch besser für biotechnologische Trennverfahren. Allerdings muß das die Partikel zurückhaltende Magnetfeld stärker sein, um die Partikel aus dem Gemisch zu entfernen. Dieses Problem kann durch Hindurchleiten der Mischung durch ein "Hochgradientenfeld" (WO 90/07380; DE 3720 844) gelöst werden.

Ein Problem stellt das ferromagnetische Material der Partikel dar, da dieses typischerweise Eisenionen abgibt, die viele biochemische Systeme stören. Man hat versucht, dieses Problem dadurch zu umgehen, daß man die Partikel mit einer Polymerschicht umgibt (WO 90/07380; DE 3720 844).

### Erfindungsgemäße technische Lösung

Erfindungsgemäß wird die Abtrennung der beim ballistischen Transfer getroffenen Zellen, in die erfolgreich die einzubringende Substanz transportiert wurde, dadurch erreicht, daß zusammen mit der einzubringenden Substanz ferromagnetische bzw. superferromagnetische Partikel in die Zellen eingebracht werden. Sofort nach dem Beschuß der Zellen mit den die Substanz und die Magnetpartikel transportierenden Mikroprojektilen werden die Zellen kurzzeitig einem Magnetfeld ausgesetzt, das die getroffenen, Magnetpartikel enthaltenen Zellen zurückhält. Nach Wegwaschen der nicht im Magnetfeld zurückgehaltenen Zellen wird das Magnetfeld entfernt und die Zellen werden je nach Aufgabenstellung weiterbehandelt.

Die beschriebene Methode ermöglicht die schnelle und effektive Abtrennung getroffener Zellen: Versuche mit Zellkulturlinien aus Säugerzellen haben ergeben, daß die Zellen die beschriebene Behandlung über Tage überleben. Versuche, bei denen fluoreszenzmarkierte DNS-Oligomere in die Zellen eingebracht wurden, haben gezeigt, daß von den zurückgehaltenen lebenden Zellen über 90% getroffen waren, was eine enorme Verbesserung bedeutet, da bei Experimenten ohne magnetische Anreicherung höchstens 40% der überlebenden Zellen die Fluoreszenzmarkierung als Zeichen des erfolgreichen Transports trugen. wendbar. Gegebenfalls können auch die Magnetpartikel selbst als Projektile verwendet werden.

Bei dem erfindungsgemäßen Verfahren haben die verwendeten Mikroprjektile einen mittleren Durchmesser von 1 bis 3 Mikrometer. Sofern es sich bei den Mikroprojektilen um Magnetpartikel handelt, können diese mit einer biologisch verträglichen Polymerschicht umhüllt sein. Dadurch können beispielsweise bei Zellen, welche im Hinblick auf ein spezifisches Gleichgewicht zwischen positiven und negativen intrazellulären Ladungen toxische Effekte durch die intrazelluläre Abgabe von Metallionen verhindert werden. Sind die Magnetpartikel polymerbeschichtet, so kann es sich auch um superferromagnetische Magnetpartikel handeln, welche einen mittleren Durchmesse von 30 - 100 nm haben.

Die Beschleunigung der Mikroprojektile, welche bei dem erfindungsgemäßen Verfahren verwendet werden, kann durch explosionsartige Entladung von Gasdruck, vorzugsweise Heliumgasdruck, erreicht werden.

Das erfindungsgemäße Verfahren eignet sich auch zur Anwendung an eukaryonten Zellen, so auch Säugerzellen, welche menschlichen Ursprungs sein können. Zudem ist es möglich, dass es sich bei den verwendeten Zellen um Zellen des humanen Immunsystems oder von humanen Tumoren handelt, welche dem Körper entnommen wurden .Das erfindungsgemäße Verfahren kann aber auch mit Pflanzenzellen durchgeführt werden.

Besondere Bedeutung hat die beschriebene Methode vor allem in der Behandlung von Krankheiten mit molekularbiologischen Ansätzen. Bis heute steht dabei die Behandlung von Krebspatienten im Mittelpunkt des Interesses. Der Ansatz beruht auf der Induktion einer gegen die Tumorzellen gerichteten Immunantwort durch Signalmoleküle des Immunsystems. Man verändert entweder Tumorzellen derart, daß sie diese Signalmoleküle (Cytokine, MHC-Oberflächenmarker) exprimieren, um durch ihr verändertes immunologisches transfizierte Zellen zurückzugeben. Nichttransfizierte Zellen werden während der Inkubationszeit die transfizierten überwachsen. Jede Verdünnung der transfizierten Zellen mit nichttransfizierten Zellen führt zu einer exponentiellen Schwächung der gewünschten Immunantwort. Damit ist die Notwendigkeit einer erfolgreichen Trennung offenkundig.

Neben der erfolgreichen Trennung von transfizierten Zellen, die aus Zellkultur erhalten wurden, eröffnet die beschriebene Methode vor allem die Möglichkeit der Transfektion von Zellen aus soliden Tumoren. Viele epidemiologisch wichtige Tumoren, wie Mammacarcinom und Coloncarcinom, lassen sich nicht oder nur schlecht in Primärkultur bringen, weil sie auf gewebespezifische Wachstumsbedingungen angewiesen sind. Die Zellen, die aus diesen Tumoren in Zellkultur wachsen, zeigen kaum Ähnlichkeit zu den nativen Tumorzellen. Dadurch wird auch die Prämisse des gentherapeutischen Ansatzes, daß transfizierte Zellen eine immunologische Reaktion auf nichttransfizierte Zellen gleichen Zelltyps auslösen sollen, haltlos. Als Alternative bietet sich die Transfektion einer Scheibe soliden Tumorgewebes an, aus dem nach Auflösen des Zellverbandes die getroffenen Zellen magnetisch abgetrennt werden.

### Beispiel:

30 µl einer Suspension von Goldpartikeln (1.6 µm Durchmesser, bezogen von Bio-Rad, Hercules, CA, USA, Konzentration der Suspension: 30 mg/ml) werden auf eine Makrocarrier-Polymerplatte (Fa. Bio-Rad) aufpippettiert. Man wartet, bis sich das Gold abgesetzt hat, und zieht den Überstand vorsichtig ab.

Auf die benetzte Fläche wird 30 µl einer 3+1 Mischung aus einer wässrigen DNS-Lösung (Konz.: 50 µg fluoresceinmarkierter Oligodesoxyribonucleotide/ml ) und einer Suspension kolloidaler Magnetpartikel (mittlerer Durchmesser: 65 nm; Miltenyi GmbH, Bergisch-Gladbach; eingesetzt, wie vom Hersteller geliefert; Konz. unbekannt; es kann vorteilhaft sein, die Magnetpartikel vor der Verwendung gegen Wasser zu dialysieren, um im Lagerungspuffer enthaltenes Natriumazid zu entfernen) pipettiert. Man wirbelt das abgesetzte Gold auf und läßt das Gemisch sich absetzen. Man zieht die überstehende Flüssigkeit ab und läßt die Goldpartikel antrocknen.

Auf eine Petrischale (3,5 cm) werden 300 µl Polylysin in der Mitte aufbebracht, 30 min stehen gelassen und mit PBS-Medium abgespült.

Zwischen 100 000 und 200 000 Zellen (Erythroleukämiezellinie K 562) werden in 300 µl RPMI-Medium (10% FKS) auf die polylysinbeschichtete Fläche der Petrischale gebracht und 10 min stehen gelassen. Man überschichtet mit 2 ml RPMI-Medium (10% FKS) und läßt 1-2 h im Brutschrank inkubieren.

Der ballistische Transfer wird an einer Biolistic PDS 1000/He (Bio-Rad, Hercules, CA, USA) nach Vorschrift des Herstellers des Apparats durchgeführt. Die verwendete Berstscheibe entspricht einem Sollbruchdruck von 1100 psi. Der Druck der Vakuumzelle beträgt 508 mm Quecksilbersäule (20 inches Hg).

Die Trennung wird auf einer MACS-Trennsäule (Miltenyi GmbH, Bergisch-Gladbach) entsprechend dem Protokoll des Herstellers durchgeführt. Es wird während des gesamten Vorganges bei 4°C gearbeitet.

Die behandelten Zellen werden in 1 ml eiskalten PBS/BSA-Medium (5mM EDTA) aufgenommen und auf die Säule gegeben, während sich diese im Magnetfeld befindet. Man wäscht mit 3 Säulenvolumina PBS/BSA-Medium (5mM EDTA) bei einer Durchflußrate von 0.3 ml/min (Ausflußnadel: Typ 25 G). Das abfließende Medium wird als Fraktion N (nichtmagnetische Fraktion) gesammelt.

Anschließend wird die Säule aus dem Magnetfeld entfernt und von unten mit 1 Säulenvolumen PBS/BSA-Medium (5mM EDTA) durchspült, um die gebundenen Zellen aufzuwirbeln.

Nach erneutem Einbringen der Säule in das Magnetfeld läßt man die Flüssigkeit ablaufen und wäscht mit 4-5 Säulenvolumina PBS/BSA-Medium (5mM EDTA) bei einer Flußrate von 0.6 ml/min (Ausflußnadel: Typ 24 G) nach.

Anschließend wird die Säule wieder aus dem Magnetfeld entfernt. Die im Magnetfeld zurückgehaltenen Zellen werden mit 1 ml PBS/BSA-Medium (5mM EDTA) in kurzen Pulsen heruntergespült. Diese Fraktion wird als Fraktion M (magnetische Fraktion) bezeichnet.

Anschließend werden die aufgefangenen Fraktionen im Durchflußcytometer (FACS) auf Fluoreszenz der in ihnen enthaltenen Zellen untersucht.

Die Fluoreszenzaktivität wurde an einem Becton-Dickinson Durchflußcytometer gemessen. Es wurden die beschossenen Zellen ohne magnetische Behandlung, die nichtmagnetische Fraktion N und die magnetische Fraktion M gemessen. Die Cytogramme im Anhang zeigen die Ergebnisse der Messungen:

U repräsentiert die nicht angereicherten Zellen nach ballistischem Transfer (unsortiert). Auf der Abszisse ist die Flureszenzaktivität der Zellen in relativen Einheiten logarithmisch aufgetragen, auf der Ordinate die Zellzahl.

In der als Zeichnung eingereichten Tabelle sind die Gesamtzahl der gezählten Fluoreszenzereignisse, sowie die den getroffenen Zellen zuzuordnende, stark fluoreszierende Teilpopulation und die sehr gering fluoreszierende, die nichtgetroffenen Zellen repräsentierende Teilpopulation in Ereigniszahlen aufgeführt, sowie deren jeweiliger relativer Anteil an der Gesamtzahl.

Gleiches gilt für die Fraktion N (nicht magnetisch rückhaltbar) und M (magnetisch rückhaltbar). Es wird die hohe Anreicherung an getroffenen Zellen in der magnetisch zurückgehaltenen Fraktion gegenüber den unsortierten Zellen deutlich.

### Beispiel 2:

### Transfektion einer Primärkultur von Melanomzellen

Melanomzellen werden in 800ml Zellkulturflaschen kultiviert. Das Medium wird abgesaugt, und die adhärenten Zellen werden mit eiskaltem PBS-Medium gewaschen. Es werden 2,5 ml Trypsin-EDTA (0,5 g Trypsin/l; 0,2 g EDTA/l; 0,85 g NaCl/l) zugegeben, und die Zellen werden bei 37 °C 2 - 5 min inkubiert. Um Zellysis zu vermeiden, wird der Vorgang beobachtet und gegebenenfalls abgebrochen. Man stoppt die Trypsinierung durch Zugabe von 25 ml eiskaltem PBS. Die Zellen werden in ein 50 ml Zentrifugenröhrchen überführt, die Zellkulturflasche mit 5 ml eiskaltem PBS ausgewaschen, und dieses wird dem Zentrifugenröhrchen hinzugefügt. Die Zellen werden bei 400 x G 7 min zentrifugiert. Der Überstand wird abgesaugt, und die Zellen werden in eiskaltem RPMI-Medium resuspendiert.

Die Zellen werden gezählt und auf 5 x 10⁶ - 1x 10⁷ Zellen pro 10 ml eingestellt. Je 5 x 10⁶ - 1x 10⁷ Zellen werden in 9,8 cm Zellkulturpetrischalen gebracht und über Nacht bei Standardbedingungen (37 °C, 5% CO₂, 90% Luftfeuchtigkeit) inkubiert.

Es verbleiben nach Absaugen des Mediums etwa 8 x 10⁶ - 1x 10⁷ Zellen adhärent in der Petrischale. Die Zellen werden mit eiskaltem PBS gewaschen, und aller Überstand wird sorgfältig entfernt. Dieser Schritt ist sehr wichtig, da jeder Flüssigkeitsfilm über den Zellen die Effizienz des nachfolgenden Transfektionsschrittes dramatisch verringert.
Der ballistische Transfer wird an einer Biolistic PDS 1000/He (Bio-Rad, Hercules, CA, USA) nach Vorschrift des Herstellers des Apparats durchgeführt. Die verwendete Berstscheibe entspricht einem Sollbruchdruck von 1550 psi. Der Druck der Vakuumzelle beträgt 508 mm Quecksilbersäule (20 inches Hg). Eine wesentliche Veränderung der verwendeten Apparatur liegt in der Verwendung eines veränderten Kopfstückes, das die gleichzeitige Transfektion einer viel größeren Zellmenge ermöglicht. Es wird die Gasdruckwelle auf sieben Makroprojektil-Halterungen verteilt, so daß sieben Geschoßgarben entstehen. Eine genaue Beschreibung der Apparatur findet sich in unserer Anmeldung "Vorrichtung zur Druckverteilung im Kopfstück einer Apparatur zum ballistischen Transfer von Zellen" vom 14.03.95 beim DPA (Aktenzeichen 195 10 696.2).

Je 30 µl einer Suspension von Goldpartikeln (1.6 µm Durchmesser, bezogen von Bio-Rad, Hercules, CA, USA, Konzentration der Suspension: 30 mg/ml) werden auf sieben Makrocarrier-Polymerplatten (Fa. Bio-Rad) aufpippettiert. Man wartet, bis sich das Gold abgesetzt hat, und zieht den Überstand vorsichtig ab.

Auf die benetzte Flächen wird 30 µl einer 3+1 Mischung aus einer wässrigen DNS-Lösung (plasmid pCMV-IL7 1 mg / ml, trägt die menschliche Interleukin 7-Sequenz unter Kontrolle eines starken Promotors aus Cytomegalovirus) und einer Suspension kolloidaler Magnetpartikel (mittlerer Durchmesser: 65 nm; Miltenyi GmbH, Bergisch-Gladbach; eingesetzt, wie vom Hersteller geliefert; Konz. unbekannt; es kann vorteilhaft sein, die Magnetpartikel vor der Verwendung gegen Wasser zu dialysieren, um im Lagerungspuffer enthaltenes Natriumazid zu entfernen) pipettiert. Man wirbelt das abgesetzte Gold auf und läßt das Gemisch sich absetzen. Man zieht die überstehende Flüssigkeit ab und läßt die Goldpartikel antrocknen.

Nach dem ballistische Transfer (1550 psi, untere Druckkammer 508 mm Hg) werden die Zellen sofort mit 3 ml eiskaltem PBS überschichtet und 5 min auf Eis gelegt, um sie von der Petrischale abzulösen. Man wäscht die Zellen durch wiederholtes Spülen von der Schale und streicht die entstandene Suspension durch ein Zellsieb (70 µm, Costar GmbH, Bodenheim, DE). Die magnetische Trennung wird durchgeführt, wie oben angegeben.

### Beispiel 3:

### Transfektion eines soliden Tumors

Tumorgewebe wird chirurgisch entnommen und auf Eis gekühlt. Nekrotische und Bindegewebsteile werden, soweit möglich, entfernt. Stücke von etwa 1 cm² Oberfläche werden herausgeschnitten, in eiskaltem PBS gewaschen und auf dem Probenhalter eines Gewebeschneiders (vibratome 1000 sectioning system; TPI, St. Louis, Missouri) mit Gewebeklebstoff fixiert. Der Tumor wird in 500 µm dicke Scheiben zerschnitten. Die Scheiben werden in eiskaltem PBS gelagert und werden so schnell wie möglich transfiziert. Der Vorgang ist mit dem in Beispiel 2 beschriebenen Vorgehen identisch. Es werden beide Seiten der Scheibe beschossen. Nach der Transfektion wird die Scheibe zwei Mal durch ein Zellsieb passiert, und die Zellen werden separiert wie beschrieben.

Da die Scheibe etwa 10 Zellschichten dick ist, aber nur die oberste Zellschicht beider Seiten getroffen werden kann, kommt man auf eine geringere Nettoausbeute transfizierter Zellen als bei Transfektion von Zellen aus Zellkultur.

### Ergebnisse der Transfektion von solidem Tumorgewebe:

### Vibratom-Schnitt-Versuche von 4 Tumorgeweben

- Tumor 1: primäres Coloncarcinom
- Tumor 2: Lebermetastase (Coloncarcinom)
- Tumor 3: Lebermetastase (Coloncarcinom)
- Tumor 4: Lebermetastase (Rectumcarcinom)

| Tumor No | Zellzahl nach Dissoziation ("ungetrennt") | | Zellzahl transfiziert ("magn. Fraktion") | | Zellzahl untransfiziert ("nichtmagn. Fraktion") | | Wiederf. in % |
|---|---|---|---|---|---|---|---|
| 1 | 1,1X10⁷ | 100% | 3,6X10⁵ | 3,3% | 7,5X10⁶ | 68,2% | 72 |
| 2 | 2,2X10⁷ | 100% | 6,9X10⁵ | 3,1% | 1.3X10⁷ | 59,1% | 62 |
| 3 | 4,1X10⁷ | 100% | 6,2X10⁵ | 1,5% | 1,9X10⁷ | 46.3% | 48 |
| 4 | 2,0X10⁷ | 100% | 7,3X10⁵ | 3,7% | 1,4X10⁷ | 70,0% | 74 |

### IL-7 ELISA:

| Tumor No | Zellzahl nach Dissoziation ("ungetrennt") | | Zellzahl transfiziert ("magn. Fraktion") | | Zellzahl untransfiziert ("nichtmagn. Fraktion") | |
|---|---|---|---|---|---|---|
| | pg IL-7·10⁻⁵ Zellen· ml⁻¹ | *pg* /*total Zellen* | pg IL-7·10⁻⁵ Zellen· ml⁻¹ | *pg* /*total Zellen* | pg IL-7·10⁻⁵ Zellen·ml⁻¹ | *pg* /*total Zellen* |
| 1 | 16 | *1,7X10*^{*8*} | 47 | *1,7X10*^{*7*} | 11,5 | *8,6X10*^{*7*} |
| 2 | 60 | *1,3X10*^{*9*} | 163 | *1,1X10*^{*8*} | 27 | *3,5X10*^{*8*} |
| 3 | 41 | *1,7X10*^{*9*} | 247 | *1,5X10*^{*8*} | 27 | *5,1X10*^{*8*} |
| 4 | 39 | *7,8X10*^{*8*} | 150 | *1,1X10*^{*8*} | 17,5 | *2,5X10*^{*8*} |

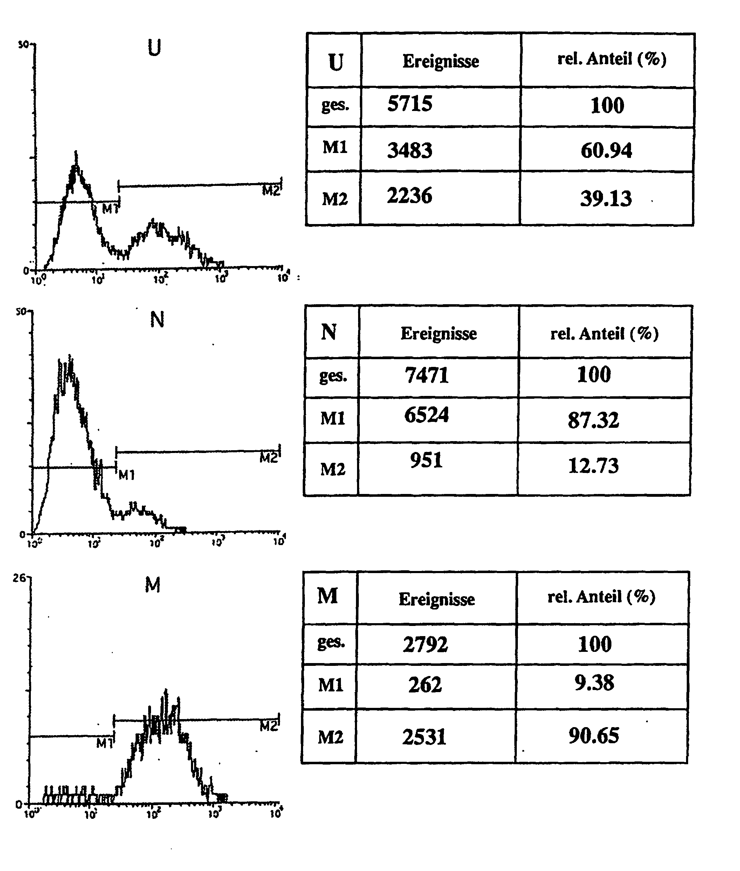

## Patentansprüche

1. Verfahren zum Einbringen von Substanzen in Zellen durch ballistischen Transfer vermittels auf die Zellen beschleunigter Mikroprojektile, an die die einzubringende Substanz adsorbiert oder auf andere Weise dauerhaft oder transient gebunden ist, oder die von der einzubringenden Substanz selbst oder ihrer Lösung gebildet werden, wobei besagte Mikroprojektile in die Zelle eindringen, ohne die Zelle zu töten, und anschließendes Abtrennen der getroffenen Zellen durch ein magnetisches Feld, **dadurch gekennzeichnet, daß** ferromagnetische Partikel zusammen mit der einzubringenden Substanz in die Zellen durch ballistischen Transfer eingebracht werden, die gesamten Zellen nach dem ballistischen Transfer in ein Magnetfeld eingebracht werden, die nicht magnetisch rückhaltbaren Zellen durch Waschen aus dem Magnetfeld entfernt werden und die im Magnetfeld zurückgehaltenen Zellen nach Entfernen desselben aufgefangen werden.

2. Verfahren nach Anspruch 1, wobei die Mikroprojektile aus einem Metall hoher Dichte, vorzugsweise Gold bestehen.

3. Verfahren nach Anspruch 1, 2 wobei die Mikroprojektile einen mittleren Durchmesser von 1 bis 3 Mikrometer haben.

4. Verfahren nach Anspruch 1 bis 3, wobei die Magnetpartikel von einer biologisch verträglichen Polymerschicht umhüllt sind.

5. Verfahren nach Anspruch 4, wobei die Magnetpartikel eine Größe von 30 - 100 nm im mittleren Durchmesser haben, superferromagnetisch sind, und das angelegte Magnetfeld ein Hochgradientenfeld ist.

6. Verfahren nach Anspruch 1 bis 5, bei dem die Mikroprojektile durch explosionsartige Entladung von Gasdruck, vorzugsweise Heliumgasdruck, auf die Zielzellen beschleunigt werden.

7. Verfahren nach Anspruch 6 bei dem die Zellen eukaryote Zellen, insbesondere Säugerzellen, sind.

8. Verfahren nach Anspruch 7, bei dem die Zellen menschliche Zellen sind.

9. Verfahren nach Anspruch 8, bei dem die Zellen aus dem menschlichen Körper entnommene Zellen des Immunsystems, oder aus dem menschlichen Körper entnommene Tumorzellen sind.

10. Verfahren nach Anspruch 9, bei dem die Zellen mit DNS-Stücken transfiziert werden, die Sequenzen für Cytokine, Oberflächenproteine oder Wachstumsfaktoren oder Teile genannter Sequenzen enthalten, oder Sequenzen, die Teile des innerzellulären Signaltransduktionsweges kodieren, oder revers komplementäre Sequenzen einer der besagten Sequenzen enthalten, wobei die genannten Sequenzen von der transfizierten Zelle transient oder permanent exprimiert werden können.

11. Verfahren nach Anspruch 1 bis 10, wobei die Zellen Pflanzenzellen sind.

## Claims

1. Method for the transfer of material into cells by ballistic transfer by means of microprojectiles that are accelerated towards said cells, onto which microprojectiles said material that is to be transferred is adsorbed or adhered to by other means permanently or transiently, or which microprojectiles are formed by the material that is to be transfered itself or its solution, where said microprojectiles penetrate the cell without killing the cell, and subsequent separation of the penetrated cells by means of a magnetic field, comprising ferromagnetic particles being transferred together with said material to be transferred by ballistic transfer, the totality of the cells being subjected to a strong magnetic field subsequent to the ballistic transfer, the cells that can not be retained by the magnetic field being removed from the magnetic field by washing, and the cells that have been retained by the magnetic field being collected after the magetic field having been removed.

2. Method according to claim 1, where the microprojectiles consist of a material of high density, preferably gold.

3. Method according to claim 1 or 2, where the microprojectiles have a median diameter of 1 to 3 micrometers

4. Method according to claim 1 to 3, where the magnetic particles are coated by a biologically compatible polymer coat.

5. Method according to claim 4, where the magnetic particles have a medium diameter of 30 -100 nm, are superferromagnetic, and the applied magnetic field is a high gradient field.

6. Method according to claim 1 to 5, where the microprojectiles are accelerated towards the target cells by means of an explosive discharge of pressurized gas, preferably pressurized helium.

7. Method according to claim 6, where the cells are eukaryotic cells, preferably mammalian cells.

8. Method according to claim 7, where the cells are human cells.

9. Method according to claim 8, where the cells are cells of the immune system that have been removed from the human body, or where the cells are tumour cells that have been removed from the human body.

10. Method according to claim 9, where the cells are transfected with DNA constructs that contain sequences encoding cytokines, surface proteins or growth factors or parts of said sequences, or sequences that encode parts of the intracellular signal transduction pathway, or reverse complementary sequences of one of said sequences, where said sequences can be expressed by the transfected cell transiently or permanently.

11. Method according to claim 1 to 10, where the cells are plant cells.

## Revendications

1. Procédé d'introduction de substances dans des cellules par transfert balistique, au moyen de micro-projectiles accélérés sur les cellules, sur lesquels la substance à introduire est adsorbée ou liée d'une autre façon de façon durable ou temporaire, ou qui sont formés de la substance à introduire elle-même ou de sa solution, dans lequel lesdits micro-projectiles pénètrent dans la cellule sans tuer la cellule, et de séparation ultérieure des cellules concernées par un champ magnétique, **caractérisé en ce que** des particules ferro-magnétiques sont introduites dans les cellules conjointement à la substance à introduire par transfert balistique, toutes les cellules, après le transfert balistique, sont introduites dans un champ magnétique, les cellules n'étant pas aptes à la retenue par voie magnétique sont éliminées du champ magnétique par lavage et les cellules retenues dans le champ magnétique sont recueillies après enlèvement de celui-ci.

2. Procédé selon la revendication 1, dans lequel les micro-projectiles se composent d'un métal de densité élevée, de préférence d'or.

3. Procédé selon la revendication 1 ou 2, dans lequel les micro-projectiles ont un diamètre moyen compris entre 1 et 3 micro-mètres.

4. Procédé selon les revendications 1 à 3, dans lequel les particules magnétiques sont enveloppées d'une couche de polymère biologiquement compatible.

5. Procédé selon la revendication 4, dans lequel les particules magnétiques ont un diamètre moyen compris entre 30 et 100 nm, sont super-ferro-magnétiques, et le champ magnétique appliqué est un champ à gradient élevé.

6. Procédé selon les revendications 1 à 5, dans lequel les micro-projectiles sont accélérés sur les cellules cibles par une décharge de pression gazeuse de type explosion, de préférence de pression d'hélium gazeux.

7. Procédé selon la revendication 6, dans lequel les cellules sont des cellules eucaryotes, en particulier des cellules de mammifères.

8. Procédé selon la revendication 7, dans lequel les cellules sont des cellules humaines.

9. Procédé selon la revendication 8, dans lequel les cellules sont des cellules du système immunitaire prélevées du corps humain ou des cellules tumorales prélevées du corps humain.

10. Procédé selon la revendication 9, dans lequel les cellules sont transfectées avec des segments d'ADN qui contiennent des séquences pour des cytokines, des protéines de surface ou des facteurs de croissance, ou des parties des séquences nommées, ou des séquences qui codent pour des parties de la voie de transduction de signal intracellulaire, ou contiennent des séquences complémentaires inverses d'une desdites séquences, les séquences nommées pouvant être exprimées de façon durable ou temporaire par la cellule transfectée.

11. Procédé selon les revendications 1 à 10, dans lequel les cellules sont des cellules végétales.
